(19) Europäisches Patentamt
European Patent Office
Office européen des brevets

(11) Veröffentlichungsnummer: **0 082 803**
**A1**

(12) # EUROPÄISCHE PATENTANMELDUNG

(21) Anmeldenummer: 82730146.6

(22) Anmeldetag: 16.12.82

(51) Int. Cl.$^3$: C 07 C 103/78
A 61 K 49/04

(30) Priorität: 18.12.81 DE 3150916

(43) Veröffentlichungstag der Anmeldung: 29.06.83 Patentblatt 83/26

(84) Benannte Vertragsstaaten: AT BE CH DE FR GB IT LI LU NL SE

(71) Anmelder: SCHERING AKTIENGESELLSCHAFT Berlin und Bergkamen
Müllerstrasse 170/178 Postfach 65 03 11
D-1000 Berlin 65(DE)

(72) Erfinder: Pfeiffer, Heinrich, Dr.
Pulfrichzeile 9
D-1000 Berlin 20(DE)

(72) Erfinder: Mützel, Wolfgang, Dr.
Weddigenweg 74
D-1000 Berlin 45(DE)

(72) Erfinder: Speck, Ulrich, Dr.
Benediktiner Strasse 50
D-1000 Berlin 28(DE)

(54) **N-Hydroxyäthylierte 2,4,6-trijodaminoisophthalsäure-bis-trihydroxybutylamide, deren Herstellung und diese enthaltende Röntgenkontrastmittel.**

(57) Die Erfindung betrifft neue N-Hydroxyäthylierte 2,4,6-trijodaminoisophthalsäure-bis-trihydroxybutylamide der allgemeinen Formel I,

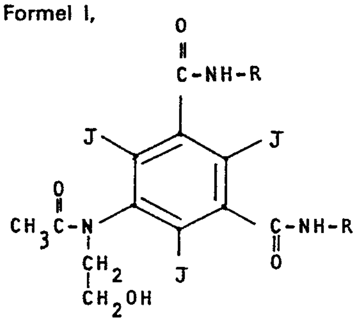

(I)

worin R einen Trihydroxybutyl-Rest darstellt.

Die Verbindungen der allgemeinen Formel I sind aufgrund ihrer ausgezeichneten Verträglichkeit und guten Wasserlöslichkeit hervorragend als schattengebende Substanzen in Röntgenkontrastmitteln zur Anwendung bei röntgendiagnostischen Untersuchungsmethoden geeignet.

EP 0 082 803 A1

Croydon Printing Company Ltd

Beschreibung

Die Erfindung betrifft neue N-Hydroxyäthylierte 2,4,6-trijodaminoisophthalsäure-bis-trihydroxybutylamide der allgemeinen Formel I,

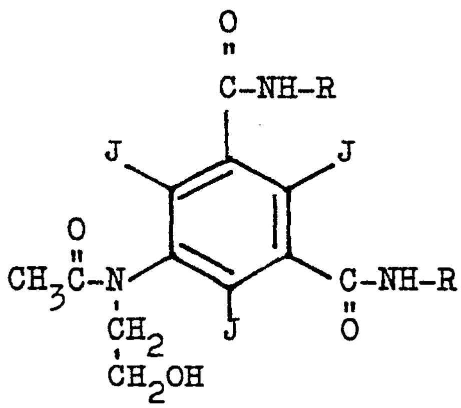

(I)

worin R einen Trihydroxybutyl-Rest darstellt.

Der Rest R als Trihydroxybutyl-Rest stellt die verschiedenen Isomeren und Enantiomeren dieses Restes dar, wie beispielsweise die 2,3,4-Trihydroxybutylreste, den 1,1,1-Trishydroxymethyl-methylrest oder die erythro- und threo-Form des 1,3,4-Trihydroxybutylrestes.

Jodhaltige, wasserlösliche Röntgenkontrastmittel werden für die Urographie, Angiographie, Myelographie, Gastrographie, Computertomographie und digitale Radiographie eingesetzt. Hinzu kommt die Darstellung von Körperhöhlen wie zum Beispiel der Gelenkhöhlen, der Gallengänge und Blase und des Pankreasganges. Seit langem ist klar, daß ein einziges Röntgenkontrastmittel dieser Vielzahl von Anwendungen nicht gerecht werden kann.

Ungenügende Verträglichkeit in der einen oder anderen Hinsicht, ungenügende Löslichkeit, Konzentration oder ungeeignete pharmakokinetische Eigenschaften schließen die Anwendung der heute verfügbaren Röntgenkontrastmittel in einigen Indikationen aus oder lassen jedenfalls viele Wünsche offen.

Die Entwicklung nicht-ionischer anstelle der bisher bekannten ionischen Röntgenkontrastmittel hat wesentliche Fortschritte im Hinblick auf die Verträglichkeit dieser Verbindungen gebracht, wobei besonders hervorzuheben sind: die geringe Schmerzhaftigkeit in der Angiographie, die geringe Kreislaufbeeinflussung und die geringe epileptogene Wirkung. Die inzwischen vorliegenden klinischen Erfahrungen mit nichtionischen und gut wasserlöslichen Röntgenkontrastmitteln (B. Hammer u. W. Lackner: Iopamidol, a new non-ionic hydrosoluble contrast medium for neuroradiology; Neuroradiology 19, 119-121, 1980) zeigen andererseits, daß auch die neuesten schattengebenden Substanzen, zu denen beispielsweise Iopamidol, Metrizamid und Iohexol zu zählen sind, noch nicht frei von Nebenwirkungen sind.

Daher besteht nach wie vor ein starkes Interesse an nicht-ionischen Röntgenkontrastmitteln, die neben den erforderlichen physikochemischen Eigenschaften, wie vor allem guter Wasserlöslichkeit und Stabilität unter Sterilisationsbedingungen, eine weiter verbesserte neurale Verträglichkeit aufweisen.

Durch eine strukturelle Abänderung von bekannten Kontrastmittelverbindungen aus der deutschen Offenlegungsschrift 2 726 196 ist es überraschend gelungen, neue trijodierte 5-Amino-isophthalsäure-bisamide mit überlegener neuraler Verträglicheit zu finden, die zusätzliche Vorteile im Hinblick auf zahlreiche für die Anwendung von Röntgenkontrastmitteln kritische Eigenschaften haben. Besonders hervorzuheben ist die durch umfangreiche extrem hydrophile Substituenten erzielte Verminderung der unerwünschten lipophilen Eigenschaften der neuen Kontrastmittel. Eine solche Reduktion der Lipophilie weist auf eine extrem gute Allgemeinverträglichkeit, wie sie sich durch eine höhere $DL_{50}$ nach i.v. Injektion bei Tieren und durch eine verminderte Häufigkeit von Übelkeit

und allergieartigen Reaktionen in der klinischen Anwendung darstellt.

Als vorteilhaft bei der Herstellung der neuen Verbindungen der Formel I hat sich herausgestellt, daß die Einführung umfangreicher Substituenten an dem Trijodaromaten nicht zu einem Viskositätsanstieg führte, der die Anwendung der betreffenden Substanzen sehr erschwert hätte.

In der Tabelle I werden folgende Substanzen im Hinblick auf die neurale und allgemeine Verträglichkeit verglichen:

$A_1$ = 5-[N-(2-Hydroxyäthyl)-acetamido]-2,4,6-trijod-isophthalsäure-bis-(threo-1,3,4-trihydroxy-but-2-yl)-diamid

$A_2$ = 5-[N-(2-Hydroxyäthyl)-acetamido]-2,4,6-trijod-isophthalsäure-bis-(erythro-1,3,4-trihydroxy-but-2-yl)-diamid

B = Metrizamid (DOS 20 31 724)

C = Iopamidol (DOS 25 47 789)

D = Iohexol (DOS 27 26 196).

Aus den Werten der Tabelle I ist zu entnehmen, daß die Substanz $A_1$ und $A_2$ sowohl in bezug auf die neurale als auch auf die allgemeine Verträglichkeit den Vergleichsverbindungen überlegen sind.

Die erfindungsgemäßen Verbindungen der Formel I können in geeigneter Zubereitung in üblicher Weise durch Erhitzen auf 120°C sterilisiert werden. Die geringe Viskosität dieser Lösungen ermöglicht eine rasche Injektion.

Die neuen Verbindungen der Formel I sind aufgrund ihrer guten pharmakologischen Eigenschaften als schattengebende Substanzen auf allen Anwendungsgebieten von wasserlöslichen Röntgenkontrastmitteln für die i.v. Appli-

**Tabelle I**

| Verbindung | $A_1$ | $A_2$ | B | C | D |
|---|---|---|---|---|---|
| neurale Verträglichkeit | | | | | |
| - Ratte, pericerebral $ED_{50}$, mg J/kg | 171<br>(122-207) | 188<br>(142-265) | 62<br>(54-72) | 154<br>(123-216) | 83<br>(60-123) |
| - Ratte, intrazisternal $ED_{50}$, mg J/kg | 130 | 130 | 48<br>(39-61) | 116<br>(95-157) | 67<br>(58-88) |
| allgemeine Verträglichkeit | | | | | |
| - Maus $LD_{50}$ (g J/kg) | 17 | 22 | 14 | 15 | 15 |
| Jodgehalt (%) | 45 | 45 | 48 | 49 | 46 |
| Zahl der Hydroxylgruppen/Molekül | 7 | 7 | 4 | 5 | 6 |
| Viskosität [m Pa · s] 37°C, 300 mg J/ml | 5.8 | 6.2 | 6.2 | 4.5 | 5.5 |

kation, insbesondere für die Angiographie, die Urographie, die Myelographie und die Computertomographie hervorragend geeignet. Da die neuen Röntgenkontrastmittel nicht resorbiert werden, sind sie auch für die orale Anwendung geeignet, beispielsweise zur Darstellung des Magen-Darm-Traktes.

Die Erfindung betrifft somit auch neue Röntgenkontrastmittel auf Basis von Verbindungen der allgemeinen Formel I.

Die Herstellung der neuen Röntgenkontrastmittel auf Basis der erfindungsgemäßen Verbindungen der allgemeinen Formel I erfolgt in an sich bekannter Weise, z.B. dadurch, daß man die schattengebende Substanz mit den in der Galenik üblichen Zusätzen, z.B. Stabilisatoren wie Natriumedetat, Calcium-di-natriumedetat, physiologisch verträglichen Puffern und dergleichen, in eine für die intravenöse Applikation geeignete Form bringt. Die Konzentration der neuen Röntgenkontrastmittel im wäßrigen Medium richtet sich nach der röntgendiagnostischen Methode. Die bevorzugten Konzentrationen und Dosierungen der neuen Verbindungen bewegen sich in den Bereichen von 50-400 mg J/ml für die Konzentration und 2-500 ml für die Dosierung. Besonders bevorzugt sind Konzentrationen zwischen 100-400 mg J/ml.

Die Erfindung betrifft ferner ein Verfahren zur Herstellung von Verbindungen der allgemeinen Formel I, dadurch gekennzeichnet, daß man in an sich bekannter Weise eine Verbindung der allgemeinen Formel II

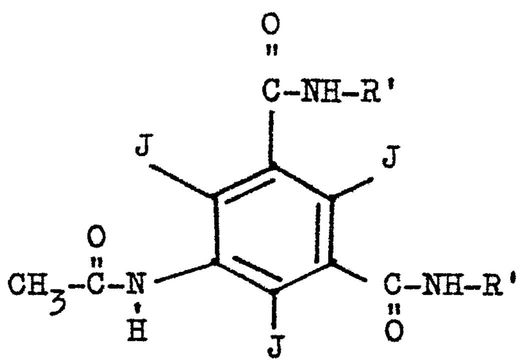

(II) ,

worin R' die obengenannte Bedeutung für R besitzt, aber im Molekül vorhandene freie Hydroxylgruppen in geschützter Form vorliegen können, mit einer Verbindung der Formel III

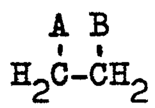

(III) ,

wobei A und B gemeinsam das Sauerstoffatom eines Oxidoringes darstellen oder

B eine Hydroxygruppe und

A ein Chlor- oder Bromatom oder eine Sulfat- oder Alkylsulfatgruppe

bedeuten, alkyliert und gewünschtenfalls anschließend geschützte Hydroxygruppen in Freiheit setzt.

Die erfindungsgemäße N-Alkylierung der 5-Acylaminogruppe erfolgt nach dem Fachmann bekannten Methoden. So kann man beispielsweise die Verbindungen der Formel II in einem geeigneten Lösungsmittel wie Methanol, Äthanol oder 1,2-Propandiol in Gegenwart von Alkalialkoholat oder Alkaliamid mit der Verbindung der Formel III, beispielsweise mit Chloräthanol oder Ethylenoxid bei einer Temperatur von Raumtemperatur bis 80°C, vozugsweise von 20° bis 60°C umsetzen.

Eine weitere Möglichkeit der Alkylierung besteht darin, die Verbindung II mit intermediär geschützten Hydroxygruppen in die Reaktion einzusetzen, wobei der Hydroxylgruppenschutz in Form eines Ketales, Acetales, Orthoesters oder als Triphenylmethylether bereits während Herstellung der Vorprodukte erfolgt ist oder erst vor der erfindungsgemäßen Reaktion durchgeführt wurde. Dies erfolgt nach üblichen Methoden durch Einführung von leicht wieder abspaltbaren Gruppen, beispielsweise durch Verätherung (z.B. Einführung des Triphenylmethylether). Der Hydroxylgruppenschutz kann auch durch Ketalisierung oder Acetalisierung, z.B. mittels Acetaldehyd, Aceton oder Dihydropyran, erreicht werden.

Die spätere Abspaltung der intermediär eingeführten Schutzgruppen unter Freisetzung der letztlich gewünschten Hydroxylgruppen erfolgt ebenfalls nach Methoden, die dem Fachmann allgemein geläufig sind. So kann die Abspaltung der Schutzgruppen ohne besondere Reaktionsstufe mit der Aufarbeitung und Isolierung der Umsetzungsprodukte erfolgen. Sie kann aber auch in üblicher Weise in einer getrennten Reaktionsstufe durchgeführt werden. Acylschutzgruppen können beispielsweise durch alkalische und Acetal-, Ketal- oder Ätherschutzgruppen durch saure Hydrolyse abgespalten werden.

Die Ausgangsverbindungen der allgemeinen Formel II sind zum Teil bekannt wie beispielsweise das 5-Acetylamino-2,4,6-trijodisophthalsäure-bis-(threo-1,3,4-trihydroxybut-2-yl)-diamid (Europäische Patentanmeldung 0033426) oder können nach Methoden, die dem Fachmann bekannt sind, hergestellt werden. Dabei geht man aus von dem bekannten 5-Acetylamino-2,4,6-trijodisophthalsäuredichlorid, das gelöst in einem geeigneten aprotischen Lösungsmittel wie beispielsweise Dimethylacetamid, mit dem gewünschten Aminobutantriol der allgemeinen Formel IV

$R'-NH_2$ (IV) umgesetzt wird,

worin R' die gleiche Bedeutung für R besitzt, jedoch einige der Hydroxygruppen in geschützter Form vorliegen können. Die für die beschriebene Reaktion benötigten Aminobutantriole der allgemeinen Formel IV sind zum Teil bekannt, wie beispielsweise 1.1.1-Trishydroxymethyl-methylamin, D,L-erythro-2-Amino-1,3,4-trihydroxybutan, cis-2,2-Dimethyl-6-hydroxy-5-amino-1,3-dioxepan oder können nach dem Fachmann bekannten Methoden hergestellt werden wie beispielsweise 2-Amino- 1-(2,2-dimethyl-1,3-dioxolan -4-yl)-ethanol, das auf folgendem Weg erhalten wird:

2-Amino-1-(2,2-dimethyl-1,3-dioxolan-4-yl)-ethanol

1) 3-Chlor-1,2,4-butantriol:

Hergestellt nach der Vorschrift von W. Reppe et al., Liebigs. Ann. Chem. 596, 137 (1955).

Ausbeute: 272 g (97 % der Theorie).
$C_4H_9ClO_3$ (140.568)

| | | | |
|---|---|---|---|
| Ber.: | 34,18 % C | 6,45 % H | 25,22 % Cl |
| Gef.: | 34,07 % C | 6,58 % H | 25,07 % Cl |

2) 2-Chlor-2-(2,2-dimethyl-1,3-dioxolan-4-yl)-ethanol

Zu einer gerührten und mit Wasser gekühlten Lösung von 272 g 3-Chlor-1,2,4-butantriol und 0,5 ml konzentrierter Schwefelsäure in einem Liter Aceton werden

258 ml 2,2-Dimethoxypropan innerhalb 2 Stunden getropft. Nach weiteren 4 Stunden ist die Umsetzung beendet. Durch Zugabe von 3,8 g Bariumhydroxid wird die Lösung neutralisiert. Es wird 30 Minuten nachgerührt, vom Feststoff abfiltriert und im Vakuum zur Trockne eingeengt. Das 2-Chlor-2-(2,2-dimethyl-1,3-dioxolan-4-yl)-ethanol wird als gelbes Öl erhalten.
Ausbeute: 340 g (94 % der Theorie).

3) 2-(2,2-Dimethyl-1,3-dioxolan-4-yl)-ethylenoxid

340 g 2-Chlor-2-(2,2-dimethyl-1,3-dioxolan-4-yl)-ethanol werden in 1,5 l absolutem Ether gelöst.
Bei 5°C werden unter intensivem Rühren insgesamt 130 g gepulvertes Kaliumhydroxid innerhalb 30 Minuten zugegeben, wobei die Temperatur durch Kühlung zwischen 5 und 15°C gehalten wird. Das Kältebad wird dann gegen ein Wasserbad (40°C) ausgetauscht, und die Suspension 2 Stunden zum gelinden Rückfluß erhitzt. Nach dem Abstellen von Heizung und Rührer beginnen sich die Phasen zu trennen. Nach Stehen über Nacht wird über Kieselgur/Sand abgesaugt. Der Rückstand wird mit Ether extrahiert. Die vereinigten Ether-Lösungen werden über eine 60 cm Vigreuxkolonne eingeengt. Der Rückstand (260 g) wird im Vakuum destilliert. Man erhält 180 g einer farblosen Flüssigkeit, die fraktioniert wird. Das 2-(2,2-Dimethyl-1,3-dioxolan-4-yl)-ethylenoxid wird bei 65°C/13 mm erhalten.
Ausbeute: 154,5 g (57 % der Theorie).
Als Nebenprodukt werden 10,8 g (4 % der Theorie 4,4-Dimethyl-3,5,8-trioxabicyclo[5.1.0]octan ($Kp_{13}$: 81°C) erhalten.

4) 2-Amino-1-(2,2-dimethyl-1,3-dioxolan-4-yl)-ethanol

Eine Lösung von 73,4 g 2-(2,2-Dimethyl-1,3-Dioxolan-4-yl)-ethylenoxid in 400 ml 25 %igem Ammoniak wird 4 Stunden auf 130°C im Autoklaven erhitzt. Die leicht gelbliche Lösung wird im Vakuum zur Trockne eingeengt, wobei der Rückstand kristallisiert. Das 2-Amino-1-(2,2-dimethyl-1,3-dioxolan-4-yl)-ethanol wird aus Ethanol/Ether umkristallisiert und zeigt einen Schmelzpunkt von 94-96°C.
Ausbeute: 52,8 g (64,2 % der Theorie).

Die Herstellung der Ausgangsverbindungen der allgemeinen Formel II wird nach der folgenden allgemeinen Arbeitsvorschrift durchgeführt:

Zu einer Lösung oder Suspension von 180 mMol freiem oder geschützten Aminobutantriol der allgemeinen Formel IV in 100 ml Dimethylacetamid wird innerhalb von 15 Minuten unter schwacher Kühlung und Rühren bei Raumtemperatur eine Lösung von 51 g (80 mMol) 5-Acetylamino-2,4,6-trijod-isophthalsäuredichlorid (DOS 20 31 724) in 100 ml Dimethylacetamid zugetropft. Anschließend werden 25,1 ml (180 mMol) Triäthylamin tropfenweise zugegeben. Nach Rühren über Nacht wird die Suspension 4 Stunden auf 50°C erwärmt, dann abgekühlt und mit 4,5 ml wäßriger konzentrierter Salzsäure angesäuert. Nach einigen Stunden wird das ausgefallene Triäthylamin-Hydrochlorid (ca. 22 g, 90 % der Theorie) abgesaugt und das Filtrat im Vakuum weitgehend eingeengt. Dieses wird mit 200 ml Wasser und 4 ml wäßriger konzentrierter Natronlauge versetzt (pH ca. 10) und einige Stunden gerührt. Bei dieser wäßrigsauren und wäßrig-alkalischen Behandlung werden in den Amidresten gegebenenfalls vorliegende Schutzgruppen im allgemeinen quantitativ abgespalten. Andernfalls sind energischere Bedingungen zu wählen oder andere übliche

BAD ORIGINAL

Abspaltungsmethoden anzuwenden. Sofern das Bisamid der allgemeinen Formel II nicht aus der wäßrigen Lösung ausfällt, wird diese mit einem Kationen- und gegebenenfalls mit einem Anionenaustauscher behandelt. Zur weiteren Reinigung kann das isolierte Produkt noch mit Äthanol gekocht werden.

Nach dieser allgemeinen Vorschrift werden erhalten:

1.) 5-Acetylamino-2,4,6-trijod-isophthalsäure-bis-(threo-1,3,4-trihydroxy-but-2-yl)-diamid

Ausbeute 51,8 g (80,4 % der Theorie); Schmp.: 258 - 260° (Zers.). Nach EP 0033426:Schmp. 246 - 247° und Ausbeute 55 % der Theorie.

2.) 5-Acetylamino-2,4,6-trijod-isophthalsäure-bis-(erythro-1,3,4-trihydroxy-but-2-yl)-diamid

Ausbeute 39 g, 61 % der Theorie; Schmp.: 275 - 276° (Zers.) unter Einsatz von 180 mMol D,L-erythro-2-Amino-1,3,4-trihydroxybutan (Kiss et al., Helv. Chim. Acta 37, 1471 [1954]) oder unter Verwendung von 180 mMol cis-2,2-Dimethyl-6-hydroxy-5-amino-1,3-dioxepan (R. Ranganathan, M. Sovak, Abstracts of Papers, 182nd ACS National Meeting 1981);

3.) 5-Acetylamino-2,4,6-trijod-isophthalsäure-bis-(2,3,4-trihydroxy-but-1-yl)-diamid

Ausbeute 47,8 g = 74 % der Theorie, Schmp.: 279 - 283° (Zers.) unter Einsatz von 180 mMol 2-Amino-1-(2,2-dimethyl-1,3-dioxolan-4-yl)-ethanol;

4.) 5-Acetylamino-2,4,6-trijod-isophthalsäure-bis-(1,1,1-tris-hydroxymethyl-methyl)-diamid

Ausbeute 23,5 g = 36,4 % der Theorie; Schmp.: 166 - 170° (Zers.) unter Einsatz von 180 mMol Trishydroxymethyl-methyl-amin. Abweichend von der allgemeinen Vorschrift wird anstelle von Triäthylamin ebenfalls Trishydroxymethyl-methylamin verwendet und der Ansatz 4 Tage auf 50° erhitzt.

Die nachfolgenden Beispiele sollen die Erfindung erläutern:

Beispiel 1

5-[N-(2-Hydroxyäthyl)-acetylamino]-2,4,6-trijod-isophthalsäure-bis-(threo-1,3,4-trihydroxy-but-2-yl)-diamid

Eine Methylatlösung aus 100 ml Methanol und 2,48 g (108 mMol) Natrium wird mit 110 ml 1,2-Propylenglykol, 40,4 g (50 mMol) 5-Acetylamino-2,4,6-trijod-isophthalsäure-bis-(threo-1,3,4-trihydroxy-but-2-yl)-diamid und zum Nachspülen mit 50 ml Methanol versetzt. Beim Rühren und Erwärmen auf 50°C entsteht eine Lösung, aus der im Vakuum das Methanol abdestilliert wird. Dann wird die Lösung mit 6,7 ml (100 mMol) 2-Chloräthanol versetzt und 5 Stunden bei 50°C weitergerührt. Nach dem Abkühlen wird die Suspension mit einem Liter Aceton versetzt und nach einer Stunde filtriert. Der natriumchloridhaltige Niederschlag wird erneut mit Aceton ausgerührt und abgesaugt. Dieses Gemisch (ca. 47 g) wird in 470 ml Wasser gelöst und über eine Säule mit 600 ml Kationenaustauscher IR 120 gegeben. Das wäßrige Eluat wird im Vakuum weitgehend eingeengt, der Rückstand mit 370 ml Wasser aufgenommen und analog mit dem Anionenaustauscher IRA 410 behandelt und aufgearbeitet. Das Auskochen des Rückstandes mit 165 ml Isopropanol liefert 24,6 g (57,7 % der Theorie Titelverbindung, Fp.: 250-254°C (Zersetzung). Jod ber. 44,73 %, gef. 44,1 %. 100 ml Wasser lösen mehr als 70 g.

Beispiel 2

5-[N-(2-Hydroxyäthyl)-acetylamino]-2,4,6-trijod-isophthalsäure-bis-(erythro-1,3,4-trihydroxy-but-2-yl)-diamid

Analog Beispiel 1 werden 40,3 g (50 mMol) 5-Acetylamino-2,4,6-trijod-isophthalsäure-bis-(erythro-1,3,4-trihydroxy-but-2-yl)-diamid hydroxyäthyliert und aufgearbeitet.

Ausbeute nach Kochen mit Isopropanol 22,6 g (53,2 % der Theorie)Titelverbindung. Fp. 292-296° (Zersetzung). Jod ber. 44,73 %, gef. 44,3 %. 100 ml Wasser lösen mehr als 70 g.

Beispiel 3

5-/N-(2-Hydroxyäthyl)-acetylamino/-2,4,6-trijod-isophthalsäure-bis-(1.1.1-trishydroxymethyl-methyl)-diamid

Analog Beispiel 1 werden 40,3 g (50 mMol) 5-Acetyl-amino-2,4,6-trijod-isophthalsäure-bis-(1.1.1-tris-hydroxy-methyl-methyl)-diamid hydroxyäthyliert und aufgearbeitet.
Ausbeute nach Kochen mit Isopropanol 23,7 g (56,9 % der Theorie) Titelverbindung. Fp. 212-218° (Zersetzung)
Jod ber. 44,73 %, gef. 44,2 %.
100 ml Wasser lösen mehr als 70 g.

Beispiel 4

5-/N-(2-Hydroxyäthyl)-acetylamino/-2,4,6-trijod-isophthalsäure-bis-(2,3,4-trihydroxy-but-1-yl)-diamid

Analog Beispiel 1 werden 40,3 g (50 mMol) 5-Acetyl-amino-2,4,6-trijod-isophthalsäure-bis-(2,3,4-trihydroxy-but-1-yl-diamid hydroxyäthyliert und aufgearbeitet.
Ausbeute nach Auskochen mit Isopropanol 27,6 g (64,7 % der Theorie) Titelverbindung, Fp. 283-287°(Zersetzung)
Jod ber. 44,73 %, gef. 44,2 %.
100 ml Wasser lösen mehr als 70 g.

**Beispiel 5**

**Herstellung einer gebrauchsfertigen wäßrigen Lösung mit einem Gehalt von 320 mg Jod/ml**

| | |
|---|---|
| 5-[N-(2-Hydroxyäthyl)-acetylamino]-2,4,6-trijod-isophthalsäure-bis-(threo-1,3,4-trihydroxy-but-2-yl)-diamid | 71,5 g |
| Calcium-dinatrium-edetat | 0,01 g |
| 2-Amino-2-(hydroxymethyl)-propan-diol-(1.3) | 0,12 g |
| 2 n Salzsäure zur Einstellung auf pH 7.1 | |
| Bidestilliertes Wasser | ad 100 ml |

Die Lösung wird in Ampullen oder Multivials abgefüllt und bei 120°C sterilisiert.

**Beispiel 6**

**Herstellung einer gebrauchsfertigen wäßrigen Lösung mit einem Jodgehalt von 380 mg J/ml**

| | |
|---|---|
| 5-[N-(2-Hydroxyäthyl)-acetylamino]-2,4,6-trijod-isophthalsäure-bis-(erythro-1,3,4-trihydroxy-but-2-yl)-diamid | 85,1 g |
| 2-Amino-2-(hydroxymethyl)-propandiol-(1,3) | 0,12 g |
| Calciumdinatriumedetat | 0,01 g |
| 2n Salzsäure zur Einstellung auf pH 7,1 | |
| Bidestilliertes Wasser | ad 100 ml |

Die Lösung wird in Ampullen oder Multivials abgefüllt und bei 120°C sterilisiert.

Beispiel 7

Herstellung einer gebrauchsfertigen wäßrigen Zubereitung für die Gastrographie mit einem Jodgehalt von 380 mg J/ml

| | |
|---|---|
| 5-/N-(2-Hydroxyäthyl)-acetylamino/-2,4,6-trijod-isophthalsäure-bis-(1.1.1-trishydroxymethyl-methyl)-diamid | 85,1 g |
| Calciumdinatriumedetat | 0,01 g |
| Anisöl | 0,14 g |
| Polyoxyäthylen-Sorbitan-oleat | 0,75 g |
| Bidestilliertes Wasser | ad 100 ml. |

Patentansprüche

1.) N-Hydroxyäthylierte 2,4,6-trijodaminoisophthalsäure-bis-trihydroxybutylamide der allgemeinen Formel I

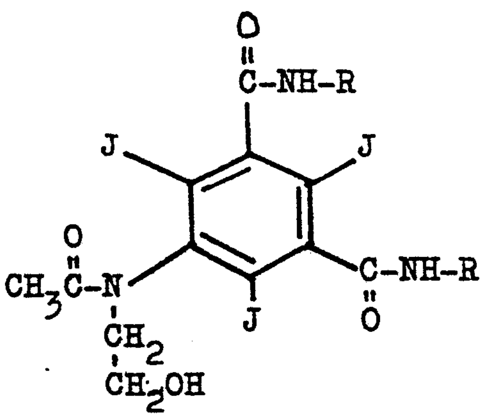

(I) ,

worin R einen Trihydroxybutyl-Rest darstellt.

2.) 5-[N-(2-Hydroxyäthyl)-acetamido]-2,4,6-trijodisophthalsäure-bis-(threo-1,3,4-trihydroxy-but-2-yl)-diamid.

3.) 5-[N-(2-Hydroxyäthyl)-acetamido]-2,4,6-trijod-isophthalsäure-bis-(erythro-1,3,4-trihydroxy-but-2-yl)-diamid.

4.) 5-[N-(2-Hydroxyäthyl)-acetamido]-2,4,6-trijod-isophthalsäure-bis-(2,3,4-trihydroxybutyl)-diamid.

5.) 5-[N-(2-Hydroxyäthyl)-acetamido]-2,4,6-trijod-isophthalsäure-bis-(1,1,1-trishydroxymethyl-methyl)-amid.

6.) Röntgenkontrastmittel enthaltend eine schattengebende Substanz gemäß Anspruch 1-5.

7.) Verfahren zur Herstellung von Verbindungen der allgemeinen Formel I,
dadurch gekennzeichnet, daß man in an sich bekannter Weise eine Verbindung der allgemeinen Formel II

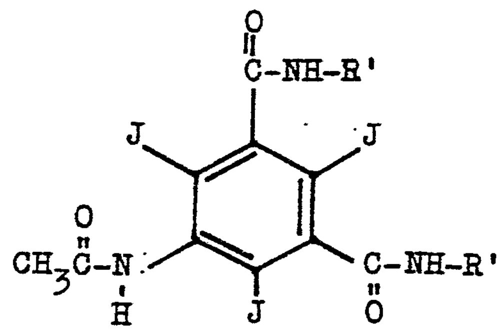

(II) ,

worin R' die obengenannte Bedeutung für R besitzt, aber im Molekül vorhandene freie Hydroxygruppen in geschützter Form vorliegen können,

mit einer Verbindung der Formel III

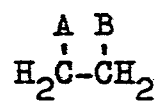

(III) ,

wobei A und B gemeinsam das Sauerstoffatom eines Oxidoringes darstellen oder

B eine Hydroxygruppe und

A ein Chlor- oder Bromatom oder eine Sulfat- oder Alkylsulfatgruppe

bedeuten,

in 5-Stellung N-alkyliert und gewünschtenfalls anschließend geschützte Hydroxylgruppen in Freiheit setzt.

Europäisches Patentamt

**0082803**

Nummer der Anmeldung

# EUROPÄISCHER RECHERCHENBERICHT

EP 82 73 0146

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 3) |
|---|---|---|---|
| X | EP-A-0 015 867 (SCHERING)<br>* Ansprüche *<br>--- | 1,6,7 | C 07 C 103/78<br>A 61 K 49/04 |
| X | FR-A-2 274 282 (MALLINCKRODT)<br>* Ansprüche *<br>--- | 1,6,7 | |
| D,Y | EP-A-0 033 426 (THE REGENTS OF THE UNIVERSITY OF CALIFORNIA)<br>* Ansprüche; Beispiele *<br>--- | 1,6 | |
| D,Y | FR-A-2 354 316 (NYEGAARD)<br>* Ansprüche *<br>----- | 1,6,7 | |

RECHERCHIERTE SACHGEBIETE (Int Cl 3)

C 07 C 103/00
A 61 K 49/00

Der vorliegende Recherchenbericht wurde fur alle Patentanspruche erstellt

| Recherchenort | Abschlußdatum der Recherche | Prufer |
|---|---|---|
| DEN HAAG | 11-03-1983 | MOREAU J.M. |

KATEGORIE DER GENANNTEN DOKUMENTEN

X von besonderer Bedeutung allein betrachtet
Y von besonderer Bedeutung in Verbindung mit einer anderen Veröffentlichung derselben Kategorie
A technologischer Hintergrund
O nichtschriftliche Offenbarung
P Zwischenliteratur
T der Erfindung zugrunde liegende Theorien oder Grundsatze

E älteres Patentdokument, das jedoch erst am oder nach dem Anmeldedatum veroffentlicht worden ist
D in der Anmeldung angeführtes Dokument
L aus andern Gründen angeführtes Dokument

& Mitglied der gleichen Patentfamilie, übereinstimmendes Dokument